(19) Europäisches Patentamt
European Patent Office
Office européen des brevets

(11) **EP 3 845 234 A1**

(12) **EUROPEAN PATENT APPLICATION**

(43) Date of publication:
**07.07.2021 Bulletin 2021/27**

(51) Int Cl.:
***A61K 36/062*** *(2006.01)* ***A23L 33/00*** *(2016.01)*
***A61P 39/06*** *(2006.01)*

(21) Application number: **19220068.1**

(22) Date of filing: **30.12.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **NextFerm Technologies Ltd.**
**2069208 Yokneam Illit (IL)**

(72) Inventors:
- **Ben-Dor, Gai**
**25257 Gita (IL)**
- **Lifshitz, Yael**
**3094206 Zichron Yaakov (IL)**
- **Yosef, Avi**
**2199734 Karmiel (IL)**
- **Cohen, Tzafra**
**3452718 Haifa (IL)**
- **Haikin, Arkadiy**
**2603607 Kiryat Motzkin (IL)**
- **Fridman, Masha**
**3268341 Haifa (IL)**
- **Khutorian, Marina**
**3681142 Nesher (IL)**
- **Mor Danan, Sivan**
**2630102 Haifa (IL)**

(74) Representative: **Greiner, Elisabeth**
**df-mp Dörries Frank-Molnia & Pohlman**
**Patentanwälte Rechtsanwälte PartG mbB**
**Theatinerstraße 16**
**80333 München (DE)**

(54) **ANTIOXIDANT EXTRACTS AND COMPOSITIONS**

(57) The present invention relates to processes for preparing antioxidant liquid extracts and compositions and uses of such antioxidant liquid extracts and compositions. Also provided are novel antioxidant liquid extracts and compositions comprising astaxanthin. The antioxidant liquid extracts and compositions inter alia exhibit high antioxidant activity.

**Figure 3**

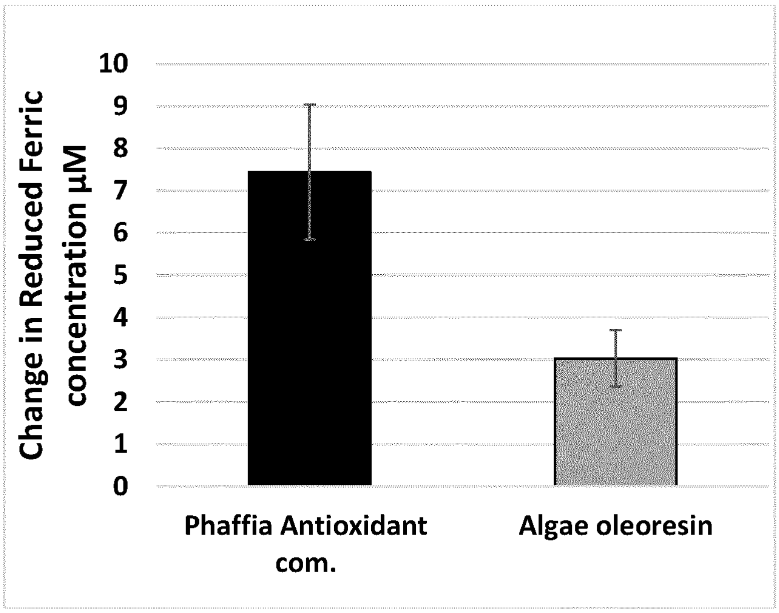




Printed by Jouve, 75001 PARIS (FR)

**Description**

**FIELD OF THE INVENTION**

The present invention relates to processes for preparing antioxidant liquid extracts and compositions and uses of such antioxidant liquid extracts and compositions. Also provided are novel antioxidant liquid extracts and compositions comprising astaxanthin. The antioxidant liquid extracts and compositions inter alia exhibit high antioxidant activity.

**BACKGROUND OF THE INVENTION**

Astaxanthin (3,3'-dihydroxy-β, β'-carotene-4,4'-dione) is a naturally-occurring lipid-soluble red oxycarotenoid pigment that is found in certain marine plants, crustaceans, fish and yeast. The red pigment colour is due to conjugated double bonds at the centre of the compound, see the planar structure of astaxanthin below:

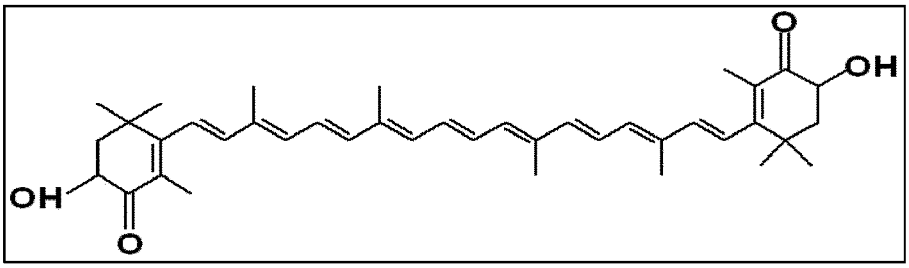

The conjugated double bonds act as a strong antioxidant by donating electrons and reacting with free radicals to convert them into more stable products, thereby terminating free radical chain reactions in a wide variety of living organisms. Astaxanthin is a stronger antioxidant than vitamin E and β-carotene. Due to its strong antioxidant activity, astaxanthin has numerous health applications, such as in cosmetology and nutraceuticals (e.g., to boost the immune system, and in mitigating the effects of ageing). There is also considerable interest in the use of astaxanthin in treating diseases such as cancer, chronic inflammatory diseases, metabolic syndrome, diabetes, diabetic nephropathy, cardiovascular diseases, gastrointestinal diseases, liver diseases, neurodegenerative diseases, eye diseases, skin diseases, exercise-induced fatigue, and male infertility.

However, due to its highly conjugated, double bond structure, astaxanthin is relatively easily oxidized and it has limited stability, both in terms of thermal stability and long-term storage stability. Oxidation of astaxanthin causes a loss of its biological activity, which may limit its use in nutraceutical and therapeutic applications. Attempts have been made to improve the stability and solubility of astaxanthin. Yet, currently available astaxanthin extracts and compositions, such as algae oleoresin oil, lack adequate stability and they are associated with further disadvantages, such as a strong and unpleasant smell and taste. Therefore, there exists a need for further astaxanthin extracts and compositions which have greater stability, higher antioxidant capacity and which are not associated with the disadvantages of the prior art, such as an unpleasant smell and taste.

The present invention fulfils these needs by providing novel antioxidant liquid extracts and compositions which can be prepared by novel processes provided herein. The antioxidant liquid extracts and compositions of the invention have greater stability and higher antioxidant capacity than antioxidant extracts and compositions of the prior art. Furthermore, the antioxidant compositions provided herein are surprisingly associated with a neutral flavour and odour, which is advantageous for a diverse range of product formulations for nutraceutical and therapeutic applications. The organoleptic superiority of the compositions of the invention enables for the first time the incorporation of astaxanthin into a variety of products beside capsulated dietary supplements, such as into drinks, powders, foods, food bars etc, without the need for flavour and odour masking; thus, without the need for additional ingredients to overcome an undesirable smell and taste. Together, the characteristics of the antioxidant liquid extracts and compositions of the invention improve and expand the nutraceutical and therapeutic applications of astaxanthin.

**BRIEF DESCRIPTION OF FIGURES**

Figure 1

Figure 1 shows the effects of an astaxanthin liquid extract according to the invention compared to algae astaxanthin oleoresin on *C. elegans* nematodes oxidative stress resistance. *C. elegans* nematodes were subjected to acute

oxidative stress by $H_2O_2$, in the presence of the antioxidant liquid extract, algae astaxanthin oleoresin or standard oil. The C. *elegans* nematodes survival rate was then measured and was calculated as percentage from initial number. **Significant P<0.001; *Significant P<0.01; NS: Not Significant.

Figure 2

Figure 2 shows the kinetics of copper-induced peroxidation of lipids in subjects' serum before (pre-dose) and following consumption of an antioxidant composition according to the invention or an algae oleoresin, 8 hours after dosing. Figure 2A plots the OD values at 245 nm monitoring the accumulation of lipid peroxidation products, dienic hydroperoxides, overtime, providing the kinetic parameters of lag time and t-max. Figure 2B quantifies the antioxidative protection effect in terms of the prolongation of the lag phase for the antioxidant composition according to the invention and the algae oleoresin (in each case, the left bar presents pre-dosing and the right bar presents 8 hours after dosing).

Figure 3

Figure 3 shows the results of a FRAP assay in the presence of an antioxidant composition according to the invention or an algae oleoresin. Healthy adult subjects were administered with either the antioxidant composition of the invention in the form of an encapsulated powder containing 50 mg astaxanthin or algae oleoresin oil containing 50 mg astaxanthin. FRAP assay was performed on plasma samples as described and calculated as "Change in Reduced Ferric concentration (μM)."

## DESCRIPTION OF THE INVENTION

### Definitions

As used herein, the term "BCAA" stands for "branched-chain amino acid". Examples include valine, leucine and isoleucine.

As used herein, the term "biomass" refers to a mass of organic matter (e.g., cells), for the purposes of the invention from a mass of *Phaffia rhodozyma* yeast.

As used herein, astaxanthin content includes free astaxanthin and various esterified astaxanthin species reported as free astaxanthin.

As used herein, "di-cis astaxanthin" means that 2 of the double bonds of the astaxanthin molecule are in their cis configuration. For example: "9,13 cis astaxanthin" means that the double bonds at positions 9 and 13 are in cis form (and not in trans form).

As used herein, the term "v/v" means volume/volume and "w/w" means "weight/weight".

As used herein, the term "ppm" refers to "parts per million" of the respective composition.

As used herein, the term "about" in reference to a value encompasses not only that precise value but also values ± 5% of that value.

The term "N.L.T." stands for "not less than".

As used herein, the term "therapy" encompasses both treatment (e.g., amelioration of symptoms) and prophylaxis (e.g., minimizing the future occurrence of symptoms).

### Processes for Preparing an Antioxidant Liquid Extract

The invention provides novel processes for preparing an antioxidant liquid extract involving the extraction of astaxanthin from *Phaffia rhodozyma.*

Specifically, the invention provides a process for preparing an antioxidant liquid extract derived from *Phaffia rhodozyma,* wherein the process comprises the following steps:

(a) contacting a *Phaffia rhodozyma* cell biomass with an organic solvent to form a mixture;
(b) performing a solid-liquid separation to remove cell debris from the mixture;
(c) evaporating the solvent from the product obtained from step (b); and
(d) heating and cooling the product obtained from step (c) to obtain said antioxidant liquid extract.

*Phaffia rhodozyma* naturally produces astaxanthin. Strains of *Phaffia rhodozyma* which overproduce astaxanthin (e.g., over 5000 μg astaxanthin per g yeast dry mass) are known to the person skilled in the art. The *Phaffia rhodozyma* cell biomass can be prepared by fermentation of *Phaffia rhodozyma* under suitable conditions known to the skilled artisan. For example, specific suitable conditions for cultivating *Phaffia rhodozyma* are incubation at about 17 to about 23°C (e.g., about 20°C), with sufficient nutrients such as a carbon source (e.g., glucose), a nitrogen source (e.g.,

$NH_4^+$), salts (e.g., $Na^+$, $K^+$, $Mg^{2+}$, $Ca^{2+}$, etc), trace elements, peptone (a water-soluble mixture of polypeptides and amino acids formed by the partial hydrolysis of protein), and cultivation at a suitable pH (e.g., an acidic pH). Sufficient nutrients can be provided by yeast growth medium as known in the art, e.g., yeast and mold "YM" medium. Typically, a yeast growth medium is a selective growth medium of acidic pH which permits the growth of yeast, while deterring growth of bacteria and other acid-intolerant organisms.

The organic solvent of step (a) can be any solvent which is capable of solubilizing at least a portion of astaxanthin. In some embodiments, the organic solvent of step (a) is ethyl acetate, acetone, ethanol, methanol, isopropanol, methylene chloride, or any mixture thereof. Typically, the organic solvent is applied at a weight biomass:volume solvent ratio of 1:2 to 1:10, preferably 1:5. Typically, step (a) is performed at a temperature of about 20 to about 80°C, preferably about 30 to about 50°C. Step (a) optionally further comprises a cell breaking step, e.g., bead milling, high pressure homogenization or equivalent mechanical breaking of the cells.

The solid-liquid separation of step (b) can be performed by filtration (e.g., by a basket centrifuge, filter press, nutsche, or the like), phase separation, centrifugation, or decantation.

Evaporating the solvent in step (c) can be performed by applying heat and optionally a vacuum. Evaporating the solvent is performed at a temperature of about 50°C or less.
Typically, the product obtained from step (c) is in the form of an oil.

The process of the invention further comprises a heating and cooling step (d). The heating performed in step (d) is done at a temperature of about 40 to about 85°C, preferably of about 45 to about 80°C, more preferably at about 50 to about 75°C. In some embodiments, the heating is performed for at least 5 hours, preferably for at least 8 hours, such as from 8 to 12 hours. Following the heating, the product is allowed to cool. Cooling is typically performed at a temperature lower than about 40°C, preferably lower than about 35°C, more preferably lower than about 30°C, for example from about 20°C to about 35°C. In some embodiments, the cooling is performed for at least 3 hours, preferably for at least 5 hours, such as for 5 to 8 hours.

The antioxidant liquid extract obtained from step (d) comprises two distinct fractions: a solid fraction comprising solid astaxanthin and an oil fraction comprising dissolved astaxanthin. The solid astaxanthin may be in any solid-state form, e.g., in the amorphous form, or in crystalline form.

The antioxidant liquid extract of the invention comprises at least about 1.5% (w/w) astaxanthin, preferably at least about 2% (w/w), more preferably at least about 2.5% (w/w), even more preferably at least about 3% (w/w), especially at least about 4% (w/w), and more especially at least about 5% (w/w) astaxanthin. The overall amount of astaxanthin comprised in the antioxidant liquid extract of the invention is also referred to as the total amount of astaxanthin.

In some embodiments, the antioxidant liquid extract of the invention comprises from about 1.5% (w/w) to about 10% (w/w) astaxanthin (total amount), preferably from about 2% (w/w) to about 8% (w/w), more preferably from about 2.5% (w/w) to about 5% (w/w) astaxanthin.

In some embodiments, at least about 10% (w/w) of the astaxanthin comprised in said antioxidant liquid extract is in the form of solid astaxanthin. Preferably at least about 20% (w/w), more preferably at least about 30% (w/w), even more preferably at least about 40% (w/w), especially at least about 50% (w/w), more especially at least about 60% (w/w), of the astaxanthin comprised in said antioxidant liquid extract is in the form of solid astaxanthin.

In one embodiment, at least about 20% (w/w) to about 90% (w/w) of the astaxanthin comprised in said antioxidant liquid extract is in the form of solid astaxanthin. Preferably about 25% (w/w) to about 80% (w/w), and more preferably from about 30% (w/w) to about 70% (w/w), of the astaxanthin comprised in said antioxidant liquid extract is in the form of solid astaxanthin. In one embodiment, about 30% (w/w) to about 60% (w/w) of the astaxanthin comprised in said antioxidant liquid extract is in the form of solid astaxanthin.

The content of dissolved astaxanthin in the clear oil out of total astaxanthin (% (w/w)) can be determined as follows:
Two samples are taken out of the antioxidant liquid extract obtained from step (d) that is kept under mixing. One sample is weighed, dissolved completely and subjected to HPLC analysis to determine the total astaxanthin content in the liquid extract. The other sample is centrifuged at 6,000 rpm for 1 min to settle the solid fraction at bottom. Clear oil liquid from the top of the centrifuge tube is carefully sampled and analyzed for astaxanthin content by HPLC.

The content of dissolved astaxanthin from total astaxanthin (% (w/w)) may be calculated as follows:

$$\text{\% dissolved astaxanthin (w/w)} = 100 * \text{\% (w/w) astaxanthin in clear oil} / \text{\% (w/w) astaxanthin in liquid extract}$$

The content of solid astaxanthin from total astaxanthin (% (w/w)) may be calculated as follows:

$$100\ \text{(w/w)} - \text{\% dissolved astaxanthin (w/w)}$$

Antioxidant Liquid Extracts

Surprisingly, the antioxidant liquid extract obtained from step (d) exhibits excellent antioxidant activity. Without wishing to be bound to theory, it is believed that the good antioxidant properties are due to the particular two-phasic composition of the extract, which comprises an exceptionally high proportion of solid astaxanthin.

Accordingly, the invention also provides an antioxidant liquid extract derived from *Phaffia rhodozyma,* wherein said liquid extract is composed of two distinct fractions: a solid fraction comprising solid astaxanthin and an oil fraction comprising dissolved astaxanthin.

The antioxidant liquid extract of the invention comprises at least about 1.5% (w/w) astaxanthin (total amount), preferably at least about 2% (w/w), more preferably at least about 2.5% (w/w), even more preferably at least about 3% (w/w), especially at least about 4% (w/w), and more especially at least about 5% (w/w) astaxanthin.

In some embodiments, the antioxidant liquid extract of the invention comprises from about 1.5% (w/w) to about 10% (w/w) astaxanthin (total amount), preferably from about 2% (w/w) to about 8% (w/w), more preferably from about 2.5% (w/w) to about 5% (w/w) astaxanthin.

In some embodiments, at least about 10% (w/w) of the astaxanthin comprised in said antioxidant liquid extract is in the form of solid astaxanthin. Preferably at least about 20% (w/w), more preferably at least about 30% (w/w), even more preferably at least about 40% (w/w), especially at least about 50% (w/w), more especially at least about 60% (w/w), of the astaxanthin comprised in said antioxidant liquid extract is in the form of solid astaxanthin.

In one embodiment, at least about 20% (w/w) to about 90% (w/w) of the astaxanthin comprised in said antioxidant liquid extract is in the form of solid astaxanthin. Preferably about 25% (w/w) to about 80% (w/w), and more preferably from about 30% (w/w) to about 70% (w/w), of the astaxanthin comprised in said antioxidant liquid extract is in the form of solid astaxanthin. In one embodiment, about 30% (w/w) to about 60% (w/w) of the astaxanthin comprised in said antioxidant liquid extract is in the form of solid astaxanthin.

In some embodiments, the solid astaxanthin comprises at least about 55% (w/w) all-trans-astaxanthin, preferably at least about 65% (w/w), more preferably at least about 70% (w/w), and most preferably at least about 80% (w/w) all-trans-astaxanthin, e.g., from about 65% (w/w) to about 95% (w/w) all-trans-astaxanthin of the total solid astaxanthin.

In some embodiments, the dissolved astaxanthin comprised in the oil fraction of the antioxidant liquid extract comprises less than about 70% (w/w) all-trans-astaxanthin, preferably less than 60% (w/w), and more preferably less than about 50% (w/w), all-trans-astaxanthin, e.g., from about 30% (w/w) to about 55% (w/w) all-trans-astaxanthin of the total dissolved astaxanthin.

In some embodiments, in both, in the solid fraction comprising solid astaxanthin and in the oil fraction comprising dissolved astaxanthin, the not-all-trans astaxanthin comprises 15cis-astaxanthin, 13cis-astaxanthin and 9cis-astaxanthin. Preferably, 15cis-astaxanthin is present in greater amounts than 13cis-astaxanthin, which is present in greater amounts than 9cis-astaxanthin.

In some embodiments, the ratio of the proportion (% w/w)) of all-trans-astaxanthin of the solid astaxanthin (% (w/w)) to the proportion of all-trans-astaxanthin (% (w/w)) of the dissolved astaxanthin (% (w/w)) is 1:1 to 10:1, preferably 1.5:1 to 5:1.

In some embodiments, the antioxidant liquid extract comprises di-cis astaxanthin in an amount of at least about 0.2% (w/w), preferably at least about 0.5% (w/w), and more preferably at least about 2% (w/w), of total astaxanthin.

In some embodiments, the solid astaxanthin comprises di-cis astaxanthin in an amount of at least about 0.1% (w/w) preferably at least about 0.2% (w/w), and more preferably at least about 0.5% (w/w), of total solid astaxanthin.

In some embodiments, the ratio of the proportion (% (w/w)) of di-cis astaxanthin in solid astaxanthin to the proportion (% (w/w)) of di-cis-astaxanthin in the dissolved astaxanthin is 1:1 to 1:50, preferably 1:1 to 1:20, more preferably 1:1 to 1:10, most preferably 1:1.5 to 1:7.

The present invention also provides an antioxidant liquid extract obtained by a process for preparing an antioxidant liquid extract derived from *Phaffia rhodozyma,* wherein the process comprises steps (a) to (d) as defined herein, together with all of their embodiments.

The antioxidant liquid extract may further be formulated into dosage forms, for example into an oral dosage form such as capsules or soft capsules or any other suitable dosage form known to the skilled artisan.

Processes for Preparing an Antioxidant Composition

The invention further provides a process for preparing an antioxidant composition wherein the process comprises the following steps:

(e) separating the solid from the liquid of an antioxidant extract derived from *Phaffia rhodozyma* by performing a solid-liquid separation;

(f) mixing the solid obtained from step (e) with water; and

(g) performing a solid-liquid separation to obtain a solid antioxidant composition.

The antioxidant extract derived from *Phaffia rhodozyma* used in step (e) can be any extract derived from *Phaffia rhodozyma.* For example, said antioxidant extract can be an antioxidant liquid extract prepared by the process as described herein.

Thus, in some embodiments, the antioxidant extract derived from *Phaffia rhodozyma* used in step (e) can be the antioxidant liquid extract prepared by a process according to steps (a) to (d) described herein.

In one embodiment, the process for preparing an antioxidant composition of the invention comprises steps (a) to (d) described herein which are performed prior to steps (e) to (g).

In one embodiment, the process for preparing an antioxidant composition comprises the following steps:

(a) contacting a *Phaffia rhodozyma* cell biomass with an organic solvent to form a mixture;
(b) performing a solid-liquid separation to remove cell debris from the mixture;
(c) evaporating the solvent from the product obtained from step (b);
(d) heating and cooling the product obtained from step (c) to obtain an antioxidant liquid extract;
(e) separating the solid from the liquid of the antioxidant liquid extract obtained in step (d);
(f) mixing the solid obtained from step (e) with water; and
(g) performing a solid-liquid separation to obtain a solid antioxidant composition.

The separating of the solid from the liquid in step (e) can be performed by filtration (e.g., by a basket centrifuge, filter press, nutsche, or the like), phase separation, centrifugation, or decantation.

In step (f), water is mixed with the solid obtained from step (e) at a weight of solid:weight of water ratio of between 1:2 to 1:50, preferably 1:10 to 1:30. The mixing is performed at a temperature of about 5 to about 95°C, preferably at a temperature of about 40 to about 90°C, and more preferably at a temperature of about 60 to about 80°C.

The solid-liquid separation of step (g) can also be performed by filtration (e.g., by a basket centrifuge, filter press, nutsche or the like), phase separation, centrifugation, or decantation. In preferred embodiments, after removal of the liquid in step (g), the solid antioxidant composition is in the form of a solid.

The solid obtained from the solid-liquid separation of step (g) may further be dried by applying heat and a vacuum (e.g., vacuum tray dryer).

The solid antioxidant composition obtained from step (g) contains very high amounts of astaxanthin. For example, said solid antioxidant composition comprises at least about 20% (w/w) of astaxanthin, preferably at least about 30% (w/w), more preferably at least about 40% (w/w), even more preferably at least about 50% (w/w), most preferably at least about 60% (w/w) of astaxanthin.

In one embodiment, the antioxidant composition comprises from about 20% (w/w) to about 60% (w/w), or from about 30% (w/w) to about 50% (w/w), or from about 40% (w/w) to about 50% (w/w) astaxanthin.

Said solid antioxidant composition obtained from step (g) exhibits very good antioxidative properties and/or olfactory properties, and/or very good stability.

## Antioxidant Compositions

Thus, the invention also provides an antioxidant composition, wherein the antioxidant composition comprises:

- astaxanthin; and
- at least one amino acid; or
- at least one metal selected from the group consisting of copper, manganese and zinc; or
- phospholipids.

Remarkably, the antioxidant compositions of the invention have greater stability and higher antioxidant capacity than antioxidant compositions of the prior art, e.g., algae oleoresin astaxanthin compositions. Furthermore, such antioxidant compositions are surprisingly associated with a neutral flavour and odour, i.e., they have improved organoleptic properties. This bears the advantage that they can be incorporated into a variety of products without the need for additional ingredients to overcome an unpleasant smell and taste which is associated with astaxanthin-containing compositions of the prior art. In addition, the compositions of the invention exhibit excellent pharmacokinetic properties and bioavailability. The compositions of the invention exhibit faster and better absorption compared to algae astaxanthin. Better absorption is demonstrated by pharmacokinetics parameters such as higher C-max (maximum plasma concentration), higher area under the plasma concentration versus time curve (AUC), and faster T-max (time at which $C_{max}$ occurs).

In some embodiments, the antioxidant composition comprises:

- astaxanthin; and
- at least one amino acid.

In some embodiments, the antioxidant composition comprises:

- astaxanthin; and
- at least one metal selected from the group consisting of copper, manganese and zinc.

In some embodiments, the antioxidant composition comprises:

- astaxanthin; and
- phospholipids.

In some embodiments, the antioxidant composition comprises:

- astaxanthin; and
- at least one amino acid; and
- at least one metal selected from the group consisting of copper, manganese and zinc.

In some embodiments, the antioxidant composition comprises:

- astaxanthin; and
- at least one amino acid; and
- phospholipids.

In some embodiments, the antioxidant composition comprises:

- astaxanthin; and
- at least one amino acid;
- at least one metal selected from the group consisting of copper, manganese and zinc; and
- phospholipids.

In some embodiments, the at least one amino acid is selected from the group consisting of valine, leucine, isoleucine, proline, glycine and phenylalanine. Preferably, the at least one amino acid is selected from the group consisting of valine, leucine, and isoleucine.

In some embodiments, the antioxidant composition comprises at least two of valine, leucine, isoleucine, proline, glycine and phenylalanine. In some embodiments, the antioxidant composition comprises at least three of valine, leucine, isoleucine, glycine, proline and phenylalanine. In some embodiments, the antioxidant composition comprises at least four of valine, leucine, isoleucine, proline, glycine and phenylalanine. Preferably, the antioxidant composition comprises valine, leucine, and isoleucine.

Typically, the at least one amino acid is comprised in the composition in an amount of from about 25 ppm to about 10000 ppm. In some embodiments, the at least one amino acid is comprised in the composition in an amount of from about 30 ppm to about 5000 ppm. Preferably, the at least one amino acid is comprised in the composition in an amount of from about 40 ppm to about 2000 ppm. More preferably, the at least one amino acid is comprised in the composition in an amount of from about 50 ppm to about 700 ppm.

In some embodiments, the antioxidant composition comprises at least two of copper, manganese and zinc. Preferably, the antioxidant composition comprises copper, manganese and zinc.

In some embodiments, the antioxidant composition comprises copper in an amount of at least about 1 ppm, or of at least about 5 ppm, or of at least about 10 ppm, or of at least about 15 ppm. Preferably, the antioxidant composition comprises copper in an amount of from about 1 ppm to about 500 ppm. More preferably, the antioxidant composition comprises copper in an amount of from about 1 ppm to about 100 ppm, or from about 5 ppm to about 100 ppm, or from about 10 ppm to about 100 ppm, or from about 15 ppm to about 100 ppm.

In some embodiments, the antioxidant composition comprises manganese in an amount of at least about 0.5 ppm, or of at least about 1 ppm, or of at least about 2 ppm, or of at least about 3 ppm, or of at least about 4 ppm, or of at least about 5 ppm. Preferably, the antioxidant composition comprises manganese in an amount of from about 0.5 ppm to about 250 ppm. More preferably, the antioxidant composition comprises manganese in an amount of from about 0.5 ppm to about 50 ppm, or from about 1 ppm to about 50 ppm, or from about 2 ppm to about 50 ppm, or from about 3 ppm to about 50 ppm, or from about 4 ppm to about 50 ppm, or from about 5 ppm to about 50 ppm.

In some embodiments, the antioxidant composition comprises zinc in an amount of at least about 10 ppm, or of at least about 20 ppm, or of at least about 30 ppm, or of at least about 40 ppm, or of at least about 50 ppm, or of at least about 60 ppm, or of at least about 70 ppm, or of at least about 80 ppm, or of at least about 90 ppm, or of at least about 100 ppm. Preferably, the antioxidant composition comprises zinc in an amount of from about 10 ppm to about 1000 ppm. More preferably, the antioxidant composition comprises zinc in an amount of from about 10 ppm to about 250 ppm, or from about 20 ppm to about 250 ppm, or from about 30 ppm to about 250 ppm, or from about 40 ppm to about 250 ppm, or from about 50 ppm to about 250 ppm, or from about 60 ppm to about 250 ppm, or from about 70 ppm to about 250 ppm, or from about 80 ppm to about 250 ppm, or from about 90 ppm to about 250 ppm, or from about 100 ppm to about 250 ppm.

In preferred embodiments, the antioxidant composition of the invention comprises astaxanthin, the amino acids valine, leucine, and isoleucine, and the metals copper, manganese and zinc; with said amino acids and said metals comprised in the composition at any of the concentration ranges disclosed herein.

In some embodiments, the antioxidant composition comprises phospholipids in an amount of at least about 0.005% (w/w), preferably of at least about 0.1% (w/w), more preferably of at least about 0.5% (w/w). Typically, about 10% (w/w) to about 90% (w/w) of the phospholipids are phosphatidylcholine.

The antioxidant compositions of the invention comprise at least about 20% (w/w) of astaxanthin, more preferably at least about 30% (w/w), even more preferably at least about 40% (w/w), especially preferably at least about 50% (w/w), even more especially preferably at least about 60% (w/w) of astaxanthin.

In some embodiments, the antioxidant composition comprises from about 20% (w/w) to about 60% (w/w), more preferably from about 30% (w/w) to about 50% (w/w), even more preferably from about 40% (w/w) to about 50% (w/w) astaxanthin.

In some embodiments, the antioxidant composition comprises di-cis astaxanthin in an amount of at least about 0.05% (w/w), at least about 0.2% (w/w), at least about 0.5% (w/w), at least about 1% (w/w), at least about 2% (w/w), and preferably at least about 3% (w/w) of total astaxanthin.

The antioxidant composition of the invention is typically in the form of a solid. In some embodiments, the solid is in the form of a powder.

The antioxidant compositions of the invention exhibit very high antioxidative activity. Antioxidative activity can be measured, for example, by several assays known in the art.

The antioxidative capacity of astaxanthin can be measured, e.g., by growing *C. elegans* nematodes in the present of astaxanthin and determining the increase in survival of said *C. elegans* subjected to acute oxidative stress in the presence of any acute oxidative stress inducer such as hydrogen peroxide ($H_2O_2$) or paraquat, e.g., about 2 mM $H_2O_2$ for about 5 hours. In some embodiments, the antioxidant compositions increase the survival of *C. elegans* nematodes under acute oxidative stress in the presence of 2 mM $H_2O_2$ by at least about 10%, preferably by at least about 20%, such as by about 20% to about 30%.

ORAC (Oxygen Radical Absorbance Capacity) is another example of a method for evaluating the antioxidant capacity of astaxanthin. This assay is an in-vitro assay measuring astaxanthin capacity to protect against singlet oxygen (Ou et. al. 2006). The assay measures the degree of inhibition of peroxy-radical-induced oxidation by the reaction between lithium molybdate and hydrogen peroxide, in the present or absence of astaxanthin. In the reaction, singlet oxygen is generated in ethanol by the molybdate-catalysed disproportionation of hydrogen peroxide at 37°C. Hydroethidine (HE), a non-fluorescent probe, is oxidized by singlet oxygen to form oxyethdium which exhibits strong fluorescence signal at 590 nm. Therefore, the inhibition of HE fluorescence in the presence of antioxidant provides an index of antioxidant capacity. The ORAC against singlet oxygen result is expressed as micromole alpha-tocopherol equivalency per gram.

Ex-vivo lag time assay is another example of a method for evaluating the antioxidant capacity of astaxanthin based on its ability as an antioxidant to delay rapid peroxidation of lipids in human serum (Pinchuk et al 2015). The assay is an ex-vivo method measuring the kinetics by continuous spectrophotometric monitoring of copper-induced lipid peroxidation followed by release of lipid oxidation products, dienic hydroperoxides, detected at 245 nm. Lag-time value, meaning the duration or the prolongation of lag phase, is the duration time during which the lipids are protected from peroxidation. This assay allows for ex vivo estimation of astaxanthin activity in serum and, therefore, the method for antioxidant evaluation can be considered as physiologically relevant. The ability of antioxidant to delay rapid peroxidation of lipids is considered a measure of the antioxidant efficiency.

Another example of a method for evaluating the antioxidant capacity of astaxanthin is the Ferric Reducing Ability of Plasma (FRAP) assay. This assay can be used to test the antioxidant capacity of astaxanthin in the plasma by measuring single electron transfer reaction. It is based on the capacity of antioxidants to reduce the ferric complex of 2, 4, 6 tripyridyl-s-triazine (Fe3+TPTZ) to the coloured ferrous complex FE2+TPTZ at pH 3.6. Interpretation of the results is based on the hypothesis that the capability of antioxidants to reduce ferric ions reflects their ability to reduce reactive oxygen species (ROS). FRAP values are obtained by comparing the absorbance change at 593 nm to a standard curve of known concentration of ferrous ions (Benzie and Strain 1996).

In some embodiments, the antioxidant liquid extract and/or the antioxidant composition has an antioxidative activity of at least 800 micromoles (μmoles) alpha-tocopherol equivalent per gram of composition. Preferably, the antioxidant liquid extract and/or the antioxidant composition has an antioxidative activity of at least 1000 micromoles (μmoles) alpha-tocopherol equivalent per gram of composition

The antioxidant compositions of the invention also exhibit high stability, in particular thermal stability. In some embodiments, the antioxidant composition shows less than 5% (w/w) degradation in astaxanthin level after incubation for 12 hours at 100°C, preferably under vacuum of 10mmHg absolute or less. Preferably, the antioxidant composition shows less than 2% (w/w) degradation in astaxanthin level after incubation for 12 hours at 100°C, preferably under vacuum of 10 mmHg absolute or less. More preferably, the antioxidant composition shows no degradation in astaxanthin level after incubation for 12 hours at 100°C, preferably under vacuum of 10 mmHg absolute or less. Degradation in astaxanthin level is defined as the reduction of astaxanthin level (% w/w) compared to baseline astaxanthin level (% w/w). It can be measured, for example, by HPLC normal phase.

In some embodiments, the antioxidant compositions of the invention are odourless and tasteless, which provides a further advantage over antioxidant compositions of the prior art.

It has been surprisingly found that the compositions obtained by the particular process of the invention provides a unique composition with excellent antioxidant and/or thermostable and/or olfactory properties. Without wishing to be bound by theory, the present inventors believe that this is due to the particular composition of the components. For example, the present inventors believe that the amino acids, and especially the branched chain amino acids (BCAAs), including leucine (Leu), isoleucine (Ile), and valine (Val), may enhance the antioxidant activity, the stability, and/orthe olfactory properties of astaxanthin. Similarly, the present inventors believe that manganese, copper and zinc may enhance the antioxidant activity, the stability, and/or the olfactory properties of astaxanthin. And the present inventors believe that phospholipids may enhance the antioxidant activity, the stability, and/or the olfactory properties of astaxanthin.

Surprisingly, the solid antioxidant composition of the invention is not only stable in dry, powder form but also when dispersed in oil.

Hence, the invention further provides an oily dispersion comprising the solid antioxidant composition in oil. The solid antioxidant composition may be comprised in said oil in an amount of about 10% (w/w), or of about 20% (w/w), or of about 30% (w/w), or of about 40% (w/w), or of about 50% (w/w), or of about 60% (w/w), or of about 70% (w/w), or any value in between.

The oil may be any oil suited for the intended purpose. For example, the oil may be an edible oil, or a pharmaceutically acceptable oil, or a cosmetically acceptable oil such as a vegetable oil. Examples of suitable vegetable oils include sunflower oil, olive oil, coconut oil, soybean oil, rapeseed oil, cannabidiol (CBD) oil and palm oil. Suitable cosmetically acceptable oils are, e.g., borage seed oil, pomegranate seed oil, evening primrose oil and the like. Such a dispersion oil can be prepared by stirring, homogenization, or pressure-homogenization of the solid antioxidant composition of the invention, and other industrial blending methods known to the person skilled in the art. Typically, the dispersion is done at a temperature of from room temperature to about 80°C, thereby providing a stabilized oilbased antioxidant composition.

The present invention also provides an antioxidant composition obtained by a process for preparing an antioxidant composition, wherein the process comprises steps (a) to (g) as defined herein, together with all of their embodiments.

The antioxidant composition may further be formulated into dosage forms, for example into an oral dosage form such as capsules or soft capsules, hard capsules, tablets or any other suitable dosage form known to the skilled artisan.

In one aspect, the invention provides an oral dosage form comprising a composition of the invention, or an antioxidant liquid extract of the invention, or an oily dispersion comprising the solid antioxidant composition of the invention. The oral dosage form is preferably a capsule or soft capsule.

## Uses of Antioxidant Extracts and Compositions

Numerous reactive oxygen species (ROS), including free radicals and singlet oxygen, are produced both in the body and by external environmental factors. When they reach the body's cells, these highly reactive species are capable of directly damaging DNA, lipids and proteins. This oxidative stress is considered to play a pivotal role in the pathogenesis of many diseases, including cardiovascular diseases, diabetes mellitus, and neurodegenerative diseases. Many of these pathologies are associated with peroxidation of oxidizable lipids in body fluids and tissues. For example, lipid peroxidation impairs membrane function and inactivates proteins and enzymes. Inhibition of lipid peroxidation is therefore be reasoned to be able to suppress the detrimental effects of oxidative stress, which may thereby help to prevent and/or treat oxidative stress-related diseases. In view of their high antioxidative activity, the antioxidant liquid extracts and compositions of the invention are capable of reducing oxidative stress, for example reducing the peroxidation of oxidizable lipids in body fluids and tissues.

Accordingly, the invention provides a use of an antioxidant composition and/or an antioxidant liquid extract provided herein as a dietary supplement, a nutritional supplement, a food supplement, a beverage supplement, feed additive such as a feed colorant or a food additive. Supplements can be in any edible form, e.g., in the form of edible candy, edible gummies, chocolates, bars or edible drinks. The antioxidant composition and/or antioxidant liquid extract can be added into such a supplement or additive as a dispersion oil. The antioxidant composition can also be added into such a supplement or additive as a powder.

Also provided herein is an antioxidant liquid extract and/or an antioxidant composition and/or oily dispersion comprising the solid antioxidant composition provided herein for use in sport, for example in preventing muscle damage and reducing joint and muscle soreness after exercise and for muscle performance and strength-endurance.

It is known that astaxanthin placed directly on the skin is capable of minimizing UV damage, the effects of air pollution and other skin stresses and insults. Accordingly, the invention also provides an antioxidant composition and/or an antioxidant liquid extract described herein for use in cosmetology. In some embodiments, the antioxidant composition and/or antioxidant liquid extract is formulated into a cosmetic composition together with one or more cosmetically acceptable excipients.

Also provided herein is an antioxidant liquid extract and/or an antioxidant composition and/or oily dispersion comprising the solid antioxidant composition provided herein for use in therapy.

Also provided herein is an antioxidant liquid extract and/or an antioxidant composition and/or oily dispersion comprising the solid antioxidant composition provided herein for use in the treatment and/or the prevention of a disease selected from a chronic inflammatory disease, a cardiovascular disease, a neurodegenerative disease, a cancer, an autoimmune disease, immune system and a liver disease.

Preferably, the liver disease is selected from alcoholic fatty liver disease (AFLD), non-alcoholic steatohepatitis (NASH) and non-alcoholic fatty liver disease (NAFLD).

Preferably, the chronic inflammatory disease is rheumatoid arthritis (RA) or atopic dermatitis.

Preferably, the cardiovascular disease is atherosclerosis and rehabilitation following heart attack.

The neurodegenerative disease may be multiple sclerosis, Alzheimer's disease, Parkinson's disease, or a prion disease. Preferably, the neurodegenerative disease is Parkinson's disease.

In some embodiments of the uses of the invention, the antioxidant liquid extract and/or the antioxidant composition is administered to a subject in need thereof as a dispersion oil. In some embodiments, antioxidant liquid extract and/or the antioxidant composition is formulated into dosage forms, for example into an oral dosage form such as capsules or soft capsules, hard capsules, tablets or any other suitable dosage form known to the skilled artisan.

In some embodiments, the antioxidant liquid extract and/or the antioxidant composition for the therapeutic uses provided herein is formulated in a pharmaceutical composition together with one or more pharmaceutically acceptable excipients.

## EXAMPLES

### Example 1: Antioxidant liquid extract derived from *Phaffia rhodozvma*

The composition of a commercially available algae oleoresin oil product containing 10% (w/w) astaxanthin was compared to an antioxidant liquid extract derived from *Phaffia rhodozyma* prepared by a process of the invention according to steps (a) to (d).

The astaxanthin contained in the algae oleoresin oil product was 100% dissolved. In contrast, the liquid extract of the invention contained about 25% of the astaxanthin as a precipitate (solid), with the remaining astaxanthin (i.e., about 75%) being dissolved in the liquid extract oil fraction.

### Example 2: Minor elements of the antioxidant composition

Table 1 presents a comparison of an element analysis of a commercially available algae oleoresin oil product containing 10% (w/w) astaxanthin and an antioxidant composition prepared by a process of the invention according to steps (a) to (g) followed by drying under vacuum.

Amino-acids levels were analysed by HPLC according to Schuster et al 1988.
Metals levels were analysed by ICP MS.
Phospholipids were analysed by quantitative $^{31}$P-NMR.

*Table 1*

| | | Algae oleoresin | Antioxidant Composition of the invention |
|---|---|---|---|
| **Astaxanthin (% w/w)** | | 10 | 60 |
| **Amino acids (ppm)** | Valine | <5 | 444 |
| | Isoleucine | <5 | 336 |
| | Leucine | <5 | 408 |
| | Proline | <5 | 152 |
| | Phenylalanine | <5 | 202 |
| | Glycine | <5 | 138 |
| **Metals (ppm)** | Copper (Cu) | 0.2 | 16.9 |
| | Manganese (Mn) | 0.14 | 3.34 |
| | Zinc (Zn) | 9.7 | 85.63 |
| **Phospholipids (% w/w)** | Phosphatidylcholine | <0.1 | 0.13 |
| | Total Phospholipids | <0.1 | 0.67 |
| **Di-cis astaxanthin (% w/w of total astaxanthin)** | | <0.01 | 0.15 |

The antioxidant composition according to the invention prepared by a process of the invention according to steps (a) to (g) contained six times more astaxanthin and over 65 times more valine, isoleucine and leucine compared to the commercially available algae oleoresin oil product. In addition, the antioxidant composition contained much greater amounts of copper, manganese and zinc metals, and a greater amount of phospholipids.

Example 3: Thermal stability

The antioxidant composition was prepared according to steps (a) to (g) of the process of the invention followed by drying in a vacuum dryer.
Additionally, an antioxidant composition dispersion oil containing 10% (w/w) astaxanthin was prepared by dispersing the antioxidant composition in sunflower oil (termed herein "antioxidant composition-10"). Briefly, the antioxidant composition prepared according to steps (a) to (g) of the process of the invention was vigorously stirred and homogenized in sunflower oil at room temperature.

The antioxidant composition, antioxidant composition-10 and algae oleoresin were tested for thermal stability. Thermal stability was determined by the reduction of astaxanthin levels as tested in HPLC normal phase compared to baseline astaxanthin levels. One (1) gram of each composition was weighed and incubated in a glass vial at 100 degrees Celsius under vacuum of 10mmHg absolute for 12 hours. The thermal stability data presented in Table 2 are the astaxanthin compositions levels (% w/w) or algae oleoresin levels (% w/w) before and after heat incubation. The results indicate that the antioxidant composition and antioxidant composition-10 have higher thermal stability compared to algae oleoresin.

*Table 2 - Thermal stability results*

| | Antioxidant composition | Antioxidant composition-10 | Algae oleoresin |
|---|---|---|---|
| Total astaxanthin % w/w T=0 hr | 68 | 10.4 | 10 |
| Total astaxanthin % w/w T=12 hr | 70 | 10.5 | 8.8 |
| Thermal stability reduction percentage | **0%** | **0%** | **12%** |
| Analytical error estimated level = 3% | | | |

**Examples 4 to 8** show the results of in vitro and ex-vivo studies demonstrating that the antioxidant liquid extract

and antioxidant compositions according to the invention have a stronger antioxidant capacity protecting against oxidative stress compared to commercially available algae astaxanthin oleoresin.

**Examples 4 and 5** show the results of in vitro studies that were performed using an antioxidant liquid extract presented in Example 1.

**Examples 6 and 7** show ex vivo studies performed in human serum samples of a clinical study following supplementation of an antioxidant composition as presented in Example 2.

**Example 8** shows the results of an ex-vivo assay performed with plasma samples of the clinical study following supplementation with an antioxidant composition as presented in Example 2.

**Example 9** shows the results of Taste and Smell Questionnaire that was provided to subjects participated in the clinical trial supplemented with an antioxidant composition as presented in Example 2.

**Example 10** shows a preparation of liquid extract.

Example 4: In vitro - Antioxidant activity of an antioxidant liquid extract in a *C. elegans* nematode model

The antioxidant activity of an antioxidant liquid extract according to the invention was compared with a commercially available algae astaxanthin oleoresin using an in vivo model of *Caenorhabditis elegans* (*C. elegans*).

For this experiment, a nematode growth medium (NGM) was prepared. Typically, NGM medium plates (1 liter of plates) are prepared as follows:

1. Combine:

NaCl 3 g
Agar 17 g
Peptone 2.5 g
Cholesterol (5 mg/ml in ethanol)
Water 975 ml

2. Autoclave
3. Maintain sterile technique and add the following, mixing after each addition:

1 M CaCl2 1 ml
1 M MgSO4 1 ml
1M PH 6 K2HPO4 25 ml.

For the in vivo model experiment, standard vegetable oil without astaxanthin served as a background control. The three samples were dissolved in DMSO and then diluted to a final concentration of 0.01 mg/mL astaxanthin in the NGM. The study control was NGM + DMSO without any antioxidants.

Synchronized hatched eggs of *C. elegans* nematodes were routinely propagated on NGM agar plates with standard *E. coli* OP50 strain as a food source. The NGM plates were prepared in the presence or absence of the different astaxanthin compositions. After 5 days of growth at 20°C, the worms were transferred to NGM plates containing 2 mM $H_2O_2$ and left for 5 hours. The incubation with 2mM $H_2O_2$ resulted in an acute oxidative stress. The nematodes were then washed, and their viability was measured. Worms were considered dead when they no longer responded to prodding. Their survival score in each feeding condition was determined.

Results are shown in Table 3 and Figure 1:
The survival percentage of *C. elegans* worms on plates containing the antioxidant liquid extract was 14% higher compared to control (p-value ≤0.001) and was 9% higher than the algae astaxanthin (which was 11% compared to control) (see Figure 1 and Table 3 below). There was no significant difference in the survival percentages between the control and standard oil, demonstrating that the antioxidant activity is contributed only by astaxanthin. This study indicates that the antioxidant liquid extract provided significantly higher resistance against acute oxidative stress compared to the algae astaxanthin oleoresin.

*Table 3*

| Sample (0.01 mg/mL astaxanthin) | % Increase Survival (vs control NGM) | P-value (vs control NGM) | P-value (vs algae oleoresin) |
|---|---|---|---|
| **Standard Oil (no astaxanthin)** | 6 | NS | <0.1 |
| **Algae oleoresin** | 11 | <0.001 | |
| **Antioxidant liquid extract** | 20 | <0.001 | <0.01 |

Example 5: An antioxidant liquid extract tested in an ORAC assay

An ORAC (Oxygen Radical Absorbance Capacity) singlet oxygen assay was performed demonstrating that an antioxidant liquid extract according to the invention has a two-fold higher capacity to protect against singlet oxygen compared to equivalent concentration of a commercially available algae astaxanthin oleoresin containing astaxanthin - see the results in Table 4.

Table 4 refers to an "alpha-tocopherol equivalent" per gram of composition (alpha-tocopherol eq/gram"), which is a measurement that is used for total antioxidant capacity of complex mixtures, such as astaxanthin compositions.

*Table 4*

| Sample | μmole alpha-tocopherol eq/gram |
|---|---|
| Algae oleoresin | 630 |
| Antioxidant liquid extract | 1,209 |

ORAC assay was performed as described in Ou, Boxin, Dejian Huang, and Maureen H. Woodill. U.S. Patent No. 7,132,296. 7 Nov. 2006.

Example 6: Ex-vivo lag time assay on human serum samples after subjects were administered an antioxidant composition in a clinical pilot study

An antioxidant composition according to the invention was tested for its antioxidant capacity to protect blood lipid peroxidation in serum samples by an ex-vivo lag time assay. Healthy adult subjects were recruited to a pilot clinical study. Following overnight fast and a high-fat breakfast, subjects were administered either the antioxidant composition of the invention in the form of an encapsulated powder containing 50 mg astaxanthin or algae oleoresin oil containing 50 mg astaxanthin. Pre-dose blood samples and post-treatment blood samples were drawn before and eight hours after dosing, respectively. Serum samples were separated from plasma and were used for the lag time assay.

Lag time assay reaction mixture: 13 ul of serum samples were incubated with 720 μM sodium citrate buffer and 100 μM copper (II) chloride to a final volume of 250 ul. Reaction mixture was added to a 96 Well UV-Star® Microplate plate and incubated in 37°C prior to reaction. Absorbance of UV light at 245 nm was monitored continuously during 5 hrs, at 3 min intervals at 37 °C using a Plate Reader (Infinite M200 PRO).

Figure 2 presents the kinetics of lipid peroxidation in subjects' serum before (pre-dose) and 8 hours following administration of the antioxidant composition or the algae oleoresin. Administration of the antioxidant composition more strongly delayed the rapid oxidation of serum lipids compared to the algae oleoresin (see Figure 2A). The two astaxanthin sources significantly differ quantitatively in their effect on lag phase (see Figure 2B). The prolongation lag time of the antioxidant composition is significantly greater than the pre-dose serum sample indicating its antioxidant activity, indicating a stronger antioxidant efficiency.

Example 7: Ex-vivo lag time assay on human serum samples after subjects were administered an antioxidant composition in a controlled clinical study

An antioxidant composition according to the invention was tested for its antioxidant capacity to protect blood lipid from peroxidation in serum samples by an ex-vivo lag time assay. Healthy adult subjects were recruited to a single-center, open-label, non-randomized, single-dose study. Following overnight fast and a high-fat breakfast, subjects were administered with antioxidant composition in the form of an encapsulated powder containing 50 mg astaxanthin. Pre-dose blood samples and post-treatment blood samples were drawn before and during 24 hours after dosing. Serum samples were separated from blood and were used for the lag time assay. Ex vivo lag time assay was conducted as described in Example 6.

Lag time assay reaction mixture: 13 ul of serum samples were incubated with 720 μM sodium citrate buffer and 100 μM copper (II) chloride to a final volume of 250 ul. Reaction mixture was added to a 96 Well UV-Star® Microplate plate and incubated in 37°C prior to reaction. Absorbance of UV light at 245 nm was monitored continuously during 5 hrs, at 3 min intervals at 37 °C using a Plate Reader (Infinite M200 PRO).

Results: Table 5 presents the lag time results of lipid peroxidation in the serum samples before (pre-dose) and following 10-12 hours after administration of the antioxidant composition. The antioxidant composition significantly delayed the rapid peroxidation of serum lipids compared to peroxidation at pre-dose time. The prolongation lag time average of the subjects included in the study is significantly greater, i.e., significantly delayed, compared to the pre-dose serum samples, indicating that the antioxidant composition affords a significant protection against lipid peroxidation and therefore a strong antioxidant activity. The highest delay was demonstrated by subject 6, who showed an increase of 38.9% above pre-dose level.

*Table 5:* Lag time percentage of the different healthy male adult subjects and lag time percentage average at 10-12 hrs after supplementation of antioxidant composition, relative to the lag time at pre-dose.

| | **Percentage of lag time at 10-12 hrs after antioxidant composition supplementation (+/- SD)** | **P value*** |
|---|---|---|
| Subject 1 | 119.7 ± 10.5 | 0.02 |
| Subject 2 | 120.2 ± 10.4 | 0.01 |
| Subject 3 | 108.9 ± 2.6 | 0.05 |
| Subject 4 | 116.4 ± 6.4 | 0.0006 |
| Subject 5 | 104.9 ± 2.8 | 0.2 |
| Subject 6 | 138.9 ± 4.4 | 0.0002 |
| Subject 7 | 128.4 ± 3.4 | 0.0001 |
| Subject 8 | 109.8 ± 4.1 | 0.03 |
| Subject 9 | 108.4 ± 13 | 0.5 |
| **Average** | **117.3 ± 10.4** | **0.0002** |
| *t-test between 100% lag time of pre-dose percentage and 10-12 hr after supplementation.<br>**Percentage of lag time 10-12 hr after antioxidant composition supplementation is calculated from pre-dose time value defined as 100%. | | |

Example 8: Ex-vivo FRAP assay in subject plasma after administration of an antioxidant composition

An antioxidant composition according to the invention was tested for its antioxidant capacity to protect against oxidation in plasma samples by an ex-vivo FRAP assay. Healthy adult subjects were recruited to a pilot clinical study. Following overnight fast and a high-fat breakfast, subjects were supplemented with either the antioxidant composition in the form of an encapsulated powder containing 50 mg astaxanthin or algae oleoresin oil containing 50 mg astaxanthin. Pre-dose blood samples and post-treatment blood samples were drawn before and 24 hours after dosing, respectively. Plasma samples were used for the FRAP assay. FRAP assay was performed as described in Benzie and Strain, Analytical Biochemistry 239, 70-76 (1996).

Figure 3 shows that the antioxidant composition had greater antioxidant capability compared to the algae composition, being more than twice as effective in increasing the amount of ferric ion reduced to ferrous ion. This demonstrates a greater ability to protect against oxidative damage in human plasma.

Example 9: Clinical study Taste and Smell Questionnaire

Twelve subjects participated in a clinical study, in which they were asked to fill in a questionnaire, within 5 minutes after dosing of the antioxidant composition in the form of an encapsulated powder containing 50 mg astaxanthin, to assess the taste and smell of the investigational product, an antioxidant composition according to the invention. The questionnaire included 3 questions and multiple answers to choose from, as detailed in Table 6.

The average value of the answer option shows that the subjects supplemented with the antioxidant composition described its smell and taste as unnoticed with no after-taste.

Table 6: Clinical study Taste and Smell Questionnaire answers and results

| Questions | | What is your impression of the product's smell? | What is your impression of the product's taste? | Did you feel an after-taste after swallowing the product? |
|---|---|---|---|---|
| Answers options: | | 1. unbearable<br>2. unpleasant<br>3. unnoticed<br>4. Pleasant | 1. unbearable<br>2. unpleasant<br>3. unnoticed<br>4. Pleasant | 1. Yes<br>2. No |
| Average | | 3 | 3 | 2 |

Example 10: Production of the liquid extract

250g of biomass from Phaffia rhodozyma (containing 0.7% w/w of astaxanthin) was mixed with 1250ml of ethyl acetate. The mixture was fed continuously, at a flow rate of about 4 liter/hr, to a bead mill (WAB, Dyno-Mill/Multi Lab, filled with 0.5mm glass beads). The temperature at the outlet of the bead mill was maintained at 30 to 45°C. The material after bead milling was filtered on a Buchner filter using a vacuum through a glass microfiber filter sheet. The cake was washed with an additional 1 liter of ethyl acetate. The filtrate obtained was evaporated in a rotary evaporator under reduced pressure, with water bath temperature of 45°C and material temperature of 25 to 35°C.

After removal of most of the solvent (when no apparent distillate was coming out), the pressure was decreased to 60mmHg absolute and the material was left for further solvent removal at about 40°C. After 4 hours under these conditions, the temperature was increased to 75°C and the material was kept at this temperature for 4 more hours.

Following the heating step above, the material was cooled to 30°C and kept at those conditions for 5 more hours. 45g of liquid extract was obtained. Liquid extract was sampled and tested and results showed that the astaxanthin concentration was 2.9% (w/w) and the content of astaxanthin in dissolved form was 31% (w/w) of total astaxanthin.

## Claims

**1.** Process for preparing an antioxidant liquid extract derived from *Phaffia rhodozyma,* wherein the process comprises the following steps:

(a) contacting a *Phaffia rhodozyma* cell biomass with an organic solvent to form a mixture;
(b) performing a solid-liquid separation to remove cell debris from the mixture;
(c) evaporating the solvent from the product obtained from step (b); and
(d) heating and cooling the product obtained from step (c) to obtain said antioxidant liquid extract.

**2.** The process for preparing an antioxidant liquid extract according to claim 1, wherein the product obtained from step (c) is heated to a temperature of about 40 to about 85°C.

**3.** The process for preparing an antioxidant liquid extract according to claim 1 or claim 2, wherein the cooling is performed at a temperature of lower than about 40°C.

**4.** Process for preparing an antioxidant composition, wherein the process comprises the following steps:

(e) separating the solid from the liquid of an antioxidant extract derived from *Phaffia rhodozyma* by performing a solid-liquid separation;
(f) mixing the solid obtained from step (e) with water; and
(g) performing a solid-liquid separation to obtain a solid antioxidant composition.

**5.** The process for preparing an antioxidant composition according to claim 4, wherein in step (f) the water is mixed with the solid obtained from step (e) at a weight of solid:weight of water ratio of between 1:2 to 1:50.

**6.** An antioxidant liquid extract derived from *Phaffia rhodozyma,* wherein said liquid extract is composed of two distinct fractions: a solid fraction comprising solid astaxanthin and an oil fraction comprising dissolved astaxanthin.

**7.** The antioxidant liquid extract according to claim 6, wherein at least about 10% (w/w) of the astaxanthin comprised in said antioxidant liquid extract is in the form of solid astaxanthin.

**8.** An antioxidant composition, wherein the antioxidant composition comprises:

- astaxanthin; and
- at least one amino acid; or
- at least one metal selected from the group consisting of copper, manganese and zinc; or
- phospholipids.

**9.** The antioxidant composition according to claim 8, wherein the at least one amino acid is selected from the group consisting of valine, leucine, isoleucine, proline, glycine and phenylalanine.

**10.** The antioxidant composition according to claim 8 or claim 9, wherein the antioxidant composition comprises copper in an amount of at least about 1 ppm; manganese in an amount of at least about 0.5 ppm; and/or zinc in an amount of at least about 10 ppm.

**11.** The antioxidant composition according to any one of claims 8 to 10, wherein the antioxidant composition is dispersed in an oil, preferably an edible oil, or wherein the antioxidant composition is in the form of a powder.

**12.** An oral dosage form comprising the antioxidant liquid extract according to claim 6 or claim 7, or the antioxidant composition according to any one of claims 8 to 11.

**13.** Use of an antioxidant liquid extract according to claim 6 or claim 7, and/or the antioxidant composition according to any one of claims 8 to 11, or the oral dosage form of claim 12 as (i) a dietary supplement, a nutritional supplement, a food supplement, a beverage supplement, or a food additive, or (ii) in sport, preferably in preventing muscle damage and reducing joint and muscle soreness after exercise and for muscle performance and strength-endurance.

**14.** The antioxidant liquid extract according to claim 6 or claim 7, and/or the antioxidant composition according to any one of claims 8 to 11, or the oral dosage form of claim 12 for use in therapy.

**15.** The antioxidant liquid extract according to claim 6 or claim 7, and/or the antioxidant composition according to any one of claims 8 to 11, or the oral dosage form of claim 12 for use in the treatment and/or prevention of a disease selected from a chronic inflammatory disease, a cardiovascular disease, a neurodegenerative disease, a cancer, an autoimmune disease, and a liver disease.

Figure 1

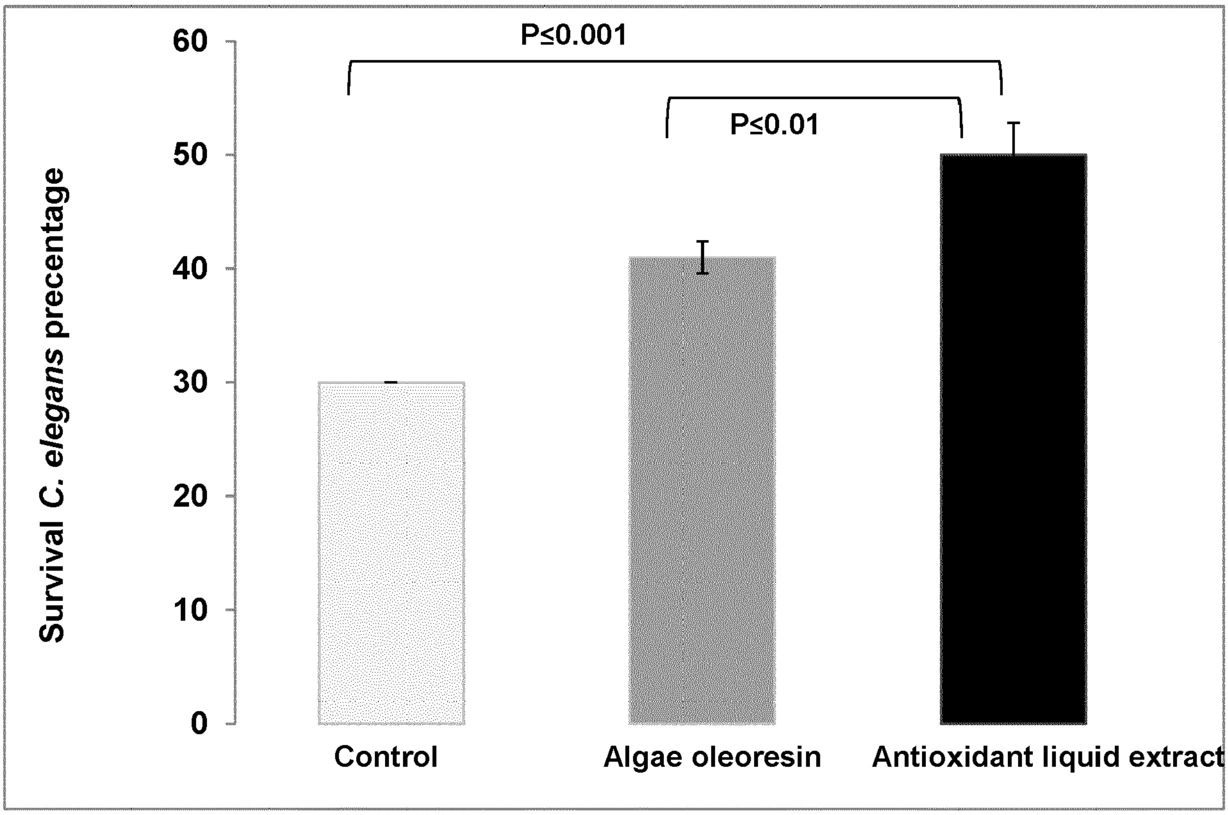
P≤0.001
P≤0.01
60
50
40
30
20
10
0
Survival *C. elegans* precentage
Control
Algae oleoresin
Antioxidant liquid extract

Figure 2A

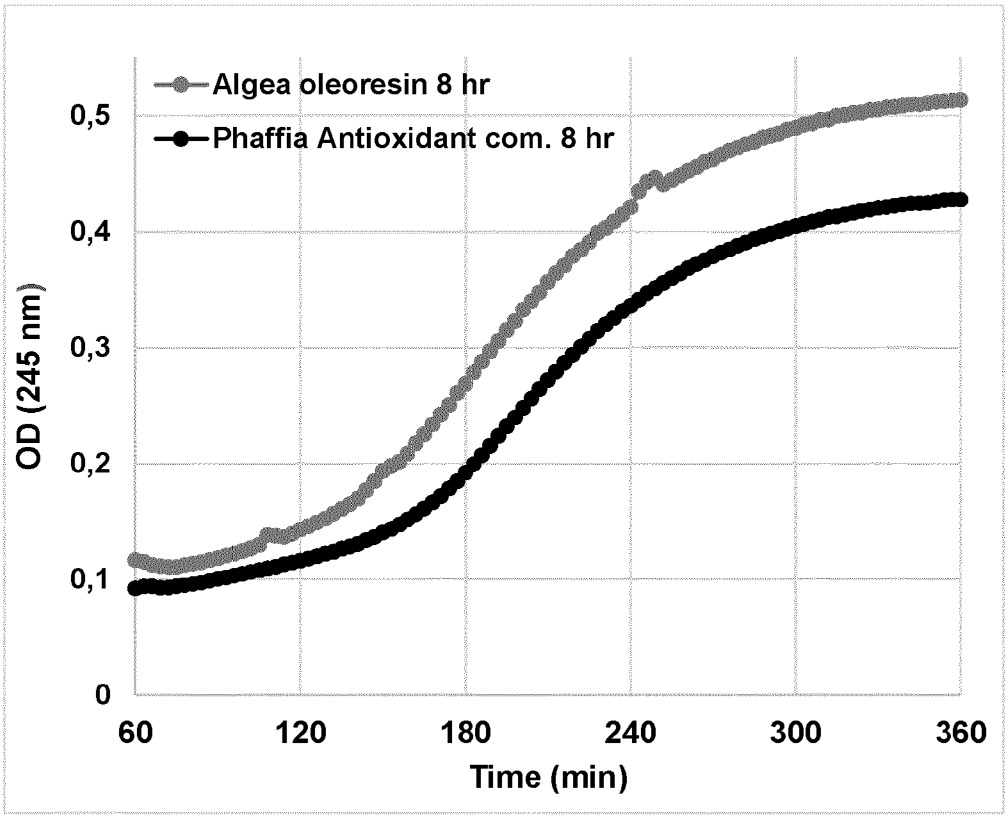


Figure 2B

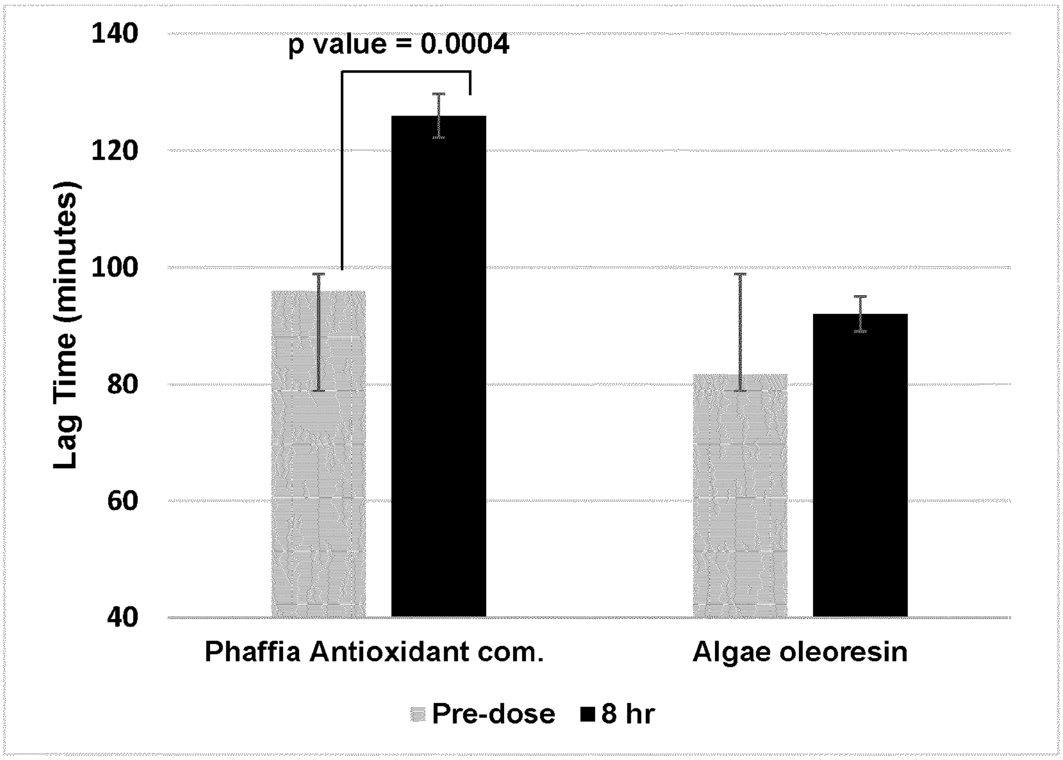

Figure 3

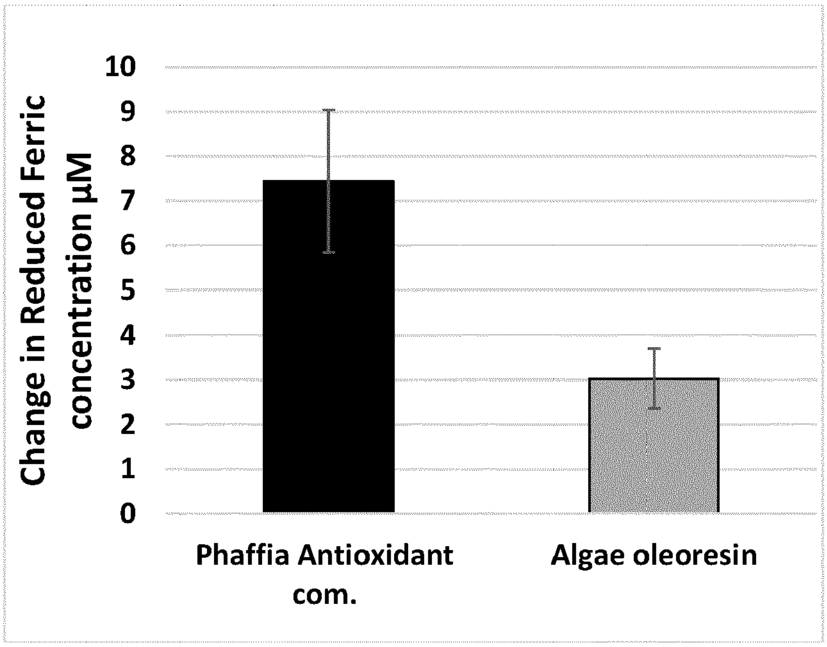
Change in Reduced Ferric concentration µM
10
9
8
7
6
5
4
3
2
1
0
Phaffia Antioxidant com.
Algae oleoresin

Europäisches Patentamt
European Patent Office
Office européen des brevets

# EUROPEAN SEARCH REPORT

Application Number
EP 19 22 0068

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/46133 A1 (FERMENTRON LTD [IL]; KAGAN MICHAEL [IL]; BRAUN SERGEI [IL]) 28 June 2001 (2001-06-28) * claims; examples * ----- | 1-15 | INV. A61K36/062 A23L33/00 A61P39/06 |
| X | XIAO JUAN LIU ET AL: "Determination of Astaxanthin in Haematococcus pluvialis by First-Order Derivative Spectrophotometry", JOURNAL OF AOAC INTERNATIONAL, vol. 94, no. 6, 1 November 2011 (2011-11-01), pages 1752-1757, XP055706191, US ISSN: 1060-3271, DOI: 10.5740/jaoacint.10-177 * table 1 * ----- | 1-15 | |

TECHNICAL FIELDS SEARCHED (IPC)

A61K
A23L
A61P

The present search report has been drawn up for all claims

1

| Place of search | Date of completion of the search | Examiner |
|---|---|---|
| Munich | 18 June 2020 | Friederich, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 22 0068

This annex lists the patent family members relating to the patent documents cited in the above-mentioned European search report. The members are as contained in the European Patent Office EDP file on
The European Patent Office is in no way liable for these particulars which are merely given for the purpose of information.

18-06-2020

| Patent document cited in search report | Publication date | Patent family member(s) | Publication date |
|---|---|---|---|
| WO 0146133 A1 | 28-06-2001 | AU 1882801 A | 03-07-2001 |
| | | CA 2395319 A1 | 28-06-2001 |
| | | EP 1240138 A1 | 18-09-2002 |
| | | GB 2358862 A | 08-08-2001 |
| | | JP 2006516293 A | 29-06-2006 |
| | | MX PA02006025 A | 23-08-2004 |
| | | US 2003044495 A1 | 06-03-2003 |
| | | WO 0146133 A1 | 28-06-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7132296 B, Ou, Boxin, Dejian Huang, and Maureen H. Woodill


### Non-patent literature cited in the description


- **BENZIE ; STRAIN.** *Analytical Biochemistry,* 1996, vol. 239, 70-76